# EUROPÄISCHE PATENTSCHRIFT

(11) **EP 2 604 410 B1**
(45) Veröffentlichungstag und Bekanntmachung des Hinweises auf die Patenterteilung: **22.10.2014**
(21) Anmeldenummer: 12197240.0
(22) Anmeldetag: 14.12.2012
(51) Int. Cl.: B29C 49/42, B29C 49/64, B29C 49/68, A61L 2/00

(54) **Vorrichtung, Getränkeabfüllanlage und Verfahren zum Umformen von Kunststoffvorformlingen**
Device for for reforming plastic blanks, beverage filling system and method
Dispositif de déformation d'ébauches en plastique, installation de remplissage de boissons et procédé

(30) Priorität: 14.12.2011 DE 102011056437
(43) Veröffentlichungstag der Anmeldung: 19.06.2013
(73) Patentinhaber: Krones AG, 93073 Neutraubling (DE)
(72) Erfinder: Laumer, Roland, 93073 Neutraubling (DE); Knott, Josef, 93073 Neutraubling (DE); Krüger, Jochen, 93073 Neutraubling (DE); Scheuren, Hans, 93073 Neutraubling (DE)
(74) Vertreter: Bittner, Bernhard

(56) Entgegenhaltungen:
- EP-A1- 2 388 129
- EP-A2- 2 366 657
- US-A1- 2003 165 400
- US-A1- 2009 317 506
- US-A1- 2010 047 120

## Beschreibung

Die Erfindung betrifft eine Vorrichtung zum Behandeln von Kunststoffbehältnissen mit einer Umformeinrichtung zum Umformen von Kunststoffvorformlingen zu den Kunststoffbehältnissen, mit einer eine Heizstrecke umfassenden Heizeinrichtung zum Erwärmen der Kunststoffvorformlinge und mit einer Sterilisationseinrichtung zum Sterilisieren der Kunststoffvorformlinge, bei welcher die Sterilisationseinrichtung eine Zuführeinrichtung umfasst, um die Kunststoffvorformlinge der Sterilisationseinrichtung zuzuführen, und bei welcher die Sterilisationseinrichtung sowohl eine Außenentkeimungseinheit, um die Kunststoffvorformlinge zumindest an ihren Außenwandungsflächen zu sterilisieren, als auch eine Innenentkeimungseinheit, um die Kunststoffvorformlinge an ihren Innenwandungsflächen zu sterilisieren, umfassen kann bzw. aufweist.

Darüber hinaus betrifft die Erfindung eine Getränkeabfüllanlage und/oder eine Getränkebehälterherstellanlage mit einer Vorrichtung zum Behandeln von Kunststoffbehältnissen. Bei den Kunststoffbehältnissen handelt es sich insbesondere um Kunststoffvorformlinge, es wäre jedoch auch eine Anwendung auf andere Kunststoffbehältnisse, wie insbesondere Kunststoffflaschen denkbar.

Auch betrifft die Erfindung ein Verfahren zum Umformen von Kunststoffvorformlingen zu Kunststoffbehältnissen, bei welchem die Kunststoffvorformlinge mittels eines gasförmigen Mediums und insbesondere Druckluft zu den Kunststoffbehältnissen expandiert werden, bei welchem die Kunststoffvorformlinge vor dem Expandieren entlang einer Heizstrecke einer Heizeinrichtung vorgewärmt werden, und bei welchem die Kunststoffvorformlinge mittels Heizstreckentransportmittel entlang der Heizstrecke von einer Heizstreckenzufuhreinrichtung zu einer Heizstreckenabfuhreinrichtung transportiert werden.

Aus dem Stand der Technik sind gattungsgemäße Vorrichtungen zum Behandeln von Kunststoffbehältnissen mit einer Umformeinrichtung zum Umformen von Kunststoffvorformlingen zu den Kunststoffbehältnissen gut bekannt.

Eine Sterilisation eines später mit Getränken zu befüllenden Kunststoffbehältnisses ist neben der eigentlichen Umformung und einem daran direkt oder später anschließenden Befüllen einer der zentralen Prozessschritte in einer zumindest teilweise aseptisch arbeitenden Vorrichtung zum Behandeln von Kunststoffbehältnissen. Die dabei zum Einsatz gelangenden Sterilisationsmöglichkeiten können insbesondere hinsichtlich der verwendbaren Entkeimungsmittel und der jeweiligen Prozessführung variieren. Anfänglich wurden nahezu ausschließend chemische Entkeimungsmittel verwendet, bei denen eine keimtötende Wirkung aufgrund chemischer Prozesse bewirkt wird. Neuere Entwicklungen grenzen sich hiervon dadurch ab, dass sie eine ionisierte Strahlung nutzen, um zumindest eine ausreichende Keimreduzierung an dem entsprechenden Kunststoffbehältnis bzw. an einem Kunststoffvorformling, aus welchem das Kunststoffbehältnis hergestellt wird, zu erzielen. Diese ionisierte Strahlung besteht in den allermeisten Anwendungen aus beschleunigten Elektronen, welche von einer entsprechenden Strahlenquelle bereitgestellt und auf das und/oder in das zu sterilisierende Kunststoffbehältnis bzw. den zu sterilisierenden Kunststoffvorformling beschleunigt werden. Ein großer Vorteil der Sterilisation mittels einer ionisierten Strahlung ist in einer Verringerung oder idealerweise in einer gänzlichen Vermeidung eines Einsatzes der chemischen Entkeimungsmittel zu sehen.

Aktuelle derzeit am Markt erhältliche Sterilisationssysteme weisen beispielsweise eine Innenentkeimungseinheit auf, die einerseits eine Elektronenerzeugungseinrichtung zum Erzeugen und Beschleunigen der Elektronen und andererseits ein Strahlfingerelement zum Leiten der Elektronen auf Innenwandungsflächen von Kunststoffbehältnissen bzw. Kunststoffvorformlingen umfassen, um die Innenwandungsflächen durch eine Öffnung der jeweiligen Kunststoffbehältnisse bzw. Kunststoffvorformlinge hindurch entkeimen zu können. Andere Sterilisationssysteme weisen Elektronenerzeugungseinrichtungen auf, mittels welchen speziell nur Außenwandungsbereiche entkeimt werden können. Daneben existieren auch Elektronenerzeugungseinrichtungen, mittels welchen Innenwandungsflächen von außen durch Wandungsbereiche der Kunststoffbehältnisse bzw. Kunststoffvorformlinge hindurch behandelt werden können.

Bevor die Kunststoffvorformlinge dem eigentlichen Expansionsprozess unterzogen werden können, müssen sie nicht nur innerhalb der jeweiligen vorgeschalteten Behandlungseinrichtung transportiert sondern auch zwischen den einzelnen Behandlungseinrichtungen übergeben werden, wodurch eine Vielzahl an Transport- Zuführ- und Übergabeeinrichtungen erforderlich ist. Beispielsweise wird ein Kunststoffvorformling einer Heizeinrichtung zuerst mittels einer Zuführeinrichtung zugeführt, dort mittels einer Transporteinrichtung zum Erwärmen entlang einer Heizstrecke an Heizelementen vorbeigeführt, um anschließend mittels einer Übergabeeinrichtung an eine Sterilisationseinrichtung übergeben zu werden. An der Sterilisationseinrichtung wird der Kunststoffvorformling mittels einer anderen Transporteinrichtung idealerweise an einer Innenentkeimungseinheit und anschließend an einer Außenentkeimungseinheit oder umgekehrt behandelt, bevor er mittels einer weiteren Übergabeeinrichtung an eine Umformeinrichtung übergeben wird. Oftmals muss der Kunststoffvorformling mehrmals mit verschiedenartigen Greifsystemen oder dergleichen gegriffen, zugeführt, übergeben, transportiert und/oder abgeführt werden, um ihn für die jeweilige Behandlung in die erforderliche Lage überführen zu können. Es versteht sich, dass aufgrund des recht hohen baulichen Aufwands nicht nur ein zeitintensiverer Transport sondern auch ein erhöhter Wartungsaufwand an einer derart komponentenreichen Vorrichtung zum Behandeln von Kunststoffbehältnissen anfällt.

Beispielsweise zeigt die Offenlegungsschrift DE 10 2008 030 156 A1 eine solche gattungsgemäße Vorrichtung und ein diesbezügliches Verfahren zum Herstellen von Kunststoffbehältnissen. Die Vorrichtung weist eine Umformungseinrichtung auf, mittels welcher Kunststoffvorformlinge zu den Kunststoffbehältnissen umgeformt werden. Die Kunststoffbehältnisse können anschließend in einer nachgeschalteten Befüllungseinrichtung insbesondere mit keimsensiblen Getränken befüllt werden, sodass zwischen der Umformungseinrichtung und der Befüllungseinrichtung eine Sterilisationseinrichtung zum Sterilisieren der Kunststoffbehältnisse angeordnet ist, um die Gefahr zu reduzieren, dass verkeimte Kunststoffbehältnisse befüllt werden, und die Getränke hierdurch ungenießbar werden. Um den eigentlichen Umformprozess günstiger durchführen zu können, werden die Kunststoffvorformlinge zuvor in einer Heizeinrichtung vorerwärmt. Dabei ist weiter vorgesehen, dass die Kunststoffvorformlinge bereits vor der Umformungseinrichtung sterilisiert werden, um insgesamt eine höhere Entkeimungssicherheit gewährleisten zu können. Insofern ist in der Offenlegungsschrift DE 10 2008 030 156 A1 vorgeschlagen, vor der Heizeinrichtung bereits eine erste Sterilisationseinrichtung vorzusehen. In diesem Zusammenhang ist erwähnt, dass diese erste oder eine weitere Sterilisationseinrichtung innerhalb der Heizeinrichtung angeordnet sein kann. Auch können beispielsweise eine Sterilisationseinrichtung vor der Heizeinrichtung, eine weitere in der Heizeinrichtung und eine weitere nach der Heizeinrichtung und vor der Umformungseinrichtung angeordnet sein.

Weitere Umformungseinrichtungen mit Sterilisationseinrichtungen werden in US 2009 317506, EP 2366657, US 2010 047120, EP 23 88 129 und US 2003 165 400 offenbart.

Die Aufgabe der Erfindung ist es, gattungsgemäße Vorrichtungen derart weiterzuentwickeln, dass deren konstruktiver Aufbau insbesondere hinsichtlich einer Sterilisationseinrichtung zum Sterilisieren von Kunststoffvorformlingen einfacher baut.

Die Aufgabe wird mit einer Vorrichtung, einer Anlage und einem Verfahren nach den Ansprüchen 1, 9 und 10 gelöst.

Idealerweise werden die an den Halteelementen der Heizstreckentransportmittel gehaltenen Kunststoffvorformlinge ohne Lageänderung bezogen auf die Kunststoffvorformling-Halteelemente der Heizstreckentransportmittel der Sterilisationseinrichtung zugeführt und dort sterilisiert.

Der Aufbau der erfindungsgemäßen Vorrichtung kann vorteilhaft weiter vereinfacht werden, wenn die Heizstreckentransportmittel eine Vielzahl an Halteelementen zum Halten der Kunststoffvorformlinge, insbesondere Dornhalteelemente zum innenseitigen Halten der Kunststoffvorformlinge, umfassen, wobei die Halteelemente bzw. die Dornhalteelemente der Heizstreckentransportmittel an der Außenentkeimungseinrichtung temporär anordenbar sind.

Derartige Halte- bzw. Dornhalteelemente sind aus dem Stand der Technik hinlänglich bekannt. Mit ihnen kann ein Kunststoffvorformling insbesondere an seinem Öffnungsbereich außen und bevorzugt innen ergriffen und gehalten werden, sodass ein vorteilhafter Einzeltransport des jeweiligen Kunststoffvorformlings gewährleistet werden kann.

Eine bevorzugte Ausführungsvariante sieht vor, dass die Sterilisationseinrichtung lediglich teilweise innerhalb der Heizeinrichtung integriert ist, wobei vorteilhaft die Außenentkeimungseinheit innerhalb der Heizstrecke und die Innenentkeimungseinheit außerhalb der Heizstrecke angeordnet sind. Insbesondere für ein Bereitstellen der Kunststoffvorformlinge an einer Außenentkeimungseinheit eignen sich bekannte Heizstreckentransportmittel sehr gut, da diese den Kunststoffvorformling von innen halten können, sodass die Gefahr einer Schattenbildung an der zu entkeimenden Außenwandungsfläche nahezu ausgeschlossen werden kann.

Der Begriff "Schattenbildung" beschreibt im Sinne der Erfindung, dass Bereiche der Außenwandungsfläche nicht oder nur unzureichend durch die Sterilisationseinrichtung entkeimt werden konnten, da sie durch Bauteile, wie etwa Halteelemente zum Haltern der Kunststoffvorformlinge, ungünstig abgedeckt sind.

Je nachdem welchen Aufbau die vorliegende Vorrichtung hat, ist es hinsichtlich einer möglichst hohen Gestaltungsfreiheit vorteilhaft, wenn die Außenentkeimungseinheit am Anfang der Heizstrecke vor einem Heizelement und nach einem Eingang der Heizeinrichtung, zwischen zwei Heizelementen innerhalb der Heizstrecke oder am Ende der Heizstrecke nach einem Heizelement und vor einer Übergabeeinrichtung am Ausgang und innerhalb der Heizeinrichtung angeordnet ist.

Der bauliche Aufwand der Vorrichtung hinsichtlich einer betriebssicheren 360° Entkeimung der Kunststoffvorformlinge kann weiter außergewöhnlich gut reduziert werden, wenn die Heizeinrichtung Mittel zum Drehen von Halte- oder Dornhalteelementen aufweist, mittels welchen die Kunststoffvorformlinge zumindest an der Außenentkeimungseinrichtung während einer Außensterilisation um eine Vertikalachse herum drehbar anordenbar sind.

Darüber hinaus ist es vorteilhaft, wenn die Heizeinrichtung eine Erzeugungseinrichtung zum Erzeugen von elektrischen Ladungsteilchen umfasst, mittels welchen die Kunststoffvorformlinge sterilisierbar sind. Durch eine derart in die Heizeinrichtung integrierte Erzeugungseinrichtung kann eine Kunststoffvorformlingsterilisation baulich weiter vereinfacht werden. Vorteilhaft weist die Entkeimungseinrichtung auch eine Beschleunigungeinrichtung zum Beschleunigen der Ladungsträger auf sowie auch ein Austrittsfenster, durch welches die Ladungsträger austreten können. Bei diesem Austrittsfenster kann es sich beispielsweise um eine Titanfolie handeln. Weiterhin ist vorteilhaft auch eine Kühleinrichtung zum Kühlen des Austrittsfensters vorgesehen, insbesondere eine Kühleinrichtung, welche das Austrittsfenster mit einem gasförmigen Medium beaufschlagt.

Bei den Ladungsträgern handelt es sich besonders bevorzugt um Elektronen, es käme jedoch auch die Verwendung anderer Ladungsträger wie beispielsweise Protonen oder alpha-Teilchen in Betracht.

Eine bevorzugte Verfahrensvariante sieht demnach auch vor, dass die Kunststoffvorformlinge innerhalb der Heizstrecke zum Sterilisieren zumindest teilweise mit Ladungsträgern bestrahlt werden.

Hierbei ist es vorteilhaft, wenn die Kunststoffvorformlinge während der Außenentkeimung um ihre Längsachse rotieren, da hierdurch auch ein zuverlässigeres Entkeimungsergebnis erzielt werden kann.

Es versteht sich, dass die Vorrichtung aufgrund der Mittel zum Drehen der Halte- oder Dornhalteelemente vorteilhafter Weise auch eine vollständige Außenentkeimung durchführen kann, wenn die Erzeugungseinrichtung lediglich nur einen Ladungsteilchen-Emitter umfasst.

Vorzugsweise ist eine derartige Erzeugungseinrichtung trotzdem mit zwei Ladungsteilchen-Emittern ausgestattet, wobei sich ein erster Ladungsteilchen-Emitter auf einer ersten Seite der Heizstreckentransportmittel und ein zweiter Ladungsteilchen-Emitter auf einer der ersten Seite gegenüberliegenden anderen Seite der Heizstreckentransportmittel angeordnet sind. Hierdurch kann eine Außenentkeimung schneller und/oder betriebssicherer durchgeführt werden. Dabei ist es möglich, dass diese einander gegenüberliegenden Ladungsteilchen-Emitter bezüglich einander entlang des Transportpfades der Behältnisse versetzt angeordnet sind. Vorteilhaft verläuft der Transportpfad der Behältnisse im Bereich der Ladungsteilchen-Emitter wenigstens abschnittsweise und bevorzugt vollständig geradlinig. Es wäre jedoch auch ein z.B. kreisförmig gekrümmter Verlauf denkbar.

Umfasst die Heizeinrichtung zumindest teilweise ein ladungsteilchenabschirmendes Gehäuse, kann die Sterilisationseinrichtung, insbesondere eine Ladungserzeugungseinrichtung der Außenentkeimungseinheit bedenkenlos in die Heizstrecke integriert werden. Daneben dient diese Abschirmung insbesondere auch zum Abschirmen der Röntgenstrahlung welche durch die besagten Ladungsteilchen bzw. deren beschleunigte Bewegung erzeugt wird.

Eine besonders betriebssichere aseptische Behandlungsumgebung kann bereits an der Heizeinrichtung geschaffen werden, wenn die Heizeinrichtung einen zusätzlichen Reinraum umfasst, in welchem die Sterilisationseinrichtung zumindest teilweise angeordnet ist. Vorzugsweise umgibt dieser Reinraum zumindest den Transportpfad der Kunststoffbehältnisse.

Darüber hinaus sieht eine besonders bevorzugte Ausführungsvariante vor, dass eine Innenentkeimungseinheit der Sterilisationseinrichtung außerhalb der Heizeinrichtung und damit beabstandet von einer innerhalb der Heizeinrichtung angeordneten Außenentkeimungseinheit der Sterilisationseinrichtung angeordnet ist, wobei insbesondere sowohl die Innenentkeimungseinheit als auch die Außenentkeimungseinheit innerhalb eines gemeinsamen Reinraums unter aseptischen Bedingungen angeordnet sind.

Ebenso ist es vorteilhaft, wenn die Heizeinrichtung ein Gehäuse aufweist, innerhalb welchem zumindest teilweise die Außenentkeimungseinheit und an dessen Ausgang die Innenentkeimungseinrichtung außerhalb des Gehäuses angeordnet ist. So kann die Heizeinrichtung sehr kompakt und kaum größer als bisher gebaut werden. Auch können hierdurch bestehende Heizeinrichtungen gegebenenfalls einfacher nachgerüstet werden.

Die Aufgabe der Erfindung wird auch von einer Getränkeabfüllanlage und/oder einer Getränkebehälterherstellanlage mit einer Vorrichtung zum Umformen von Kunststoffvorformlingen zu Kunststoffbehältnissen nach einem der hier beschriebenen Merkmale gelöst.

Außerdem wird die Aufgabe der Erfindung auch von einer Verwendung von Heizstreckentransportmitteln, mit welchen Kunststoffvorformlinge entlang der Heizstrecke einer Heizeinrichtung zum Erwärmen der Kunststoffvorformlinge bewegt werden, zum Halten der Kunststoffvorformlinge vor einer Entkeimungseinheit einer Sterilisationseinrichtung zum Sterilisieren der Kunststoffvorformlinge gelöst.

Durch eine derartige erfindungsgemäße Verwendung kann der konstruktive Aufwand zum Transportieren der Kunststoffvorformlinge besonders gering gehalten werden.

Es versteht sich, dass die vorliegende Erfindung nicht nur auf eine Heizeinrichtung zum Erwärmen von Kunststoffvorformlingen vorteilhaft angewendet werden kann, sondern auch hinsichtlich einer Heizeinrichtung für Kunststoffbehältnisse, wenn dies zweckdienlich ist.

Weitere Vorteile, Ziele und Eigenschaften vorliegender Erfindung werden anhand anliegender Zeichnung und nachfolgender Beschreibung erläutert, in welchen beispielhaft eine Getränkebehälterherstellanlage mit einer eine Sterilisationseinrichtung umfassenden Heizeinrichtung dargestellt und beschrieben ist. In der Zeichnung zeigen:

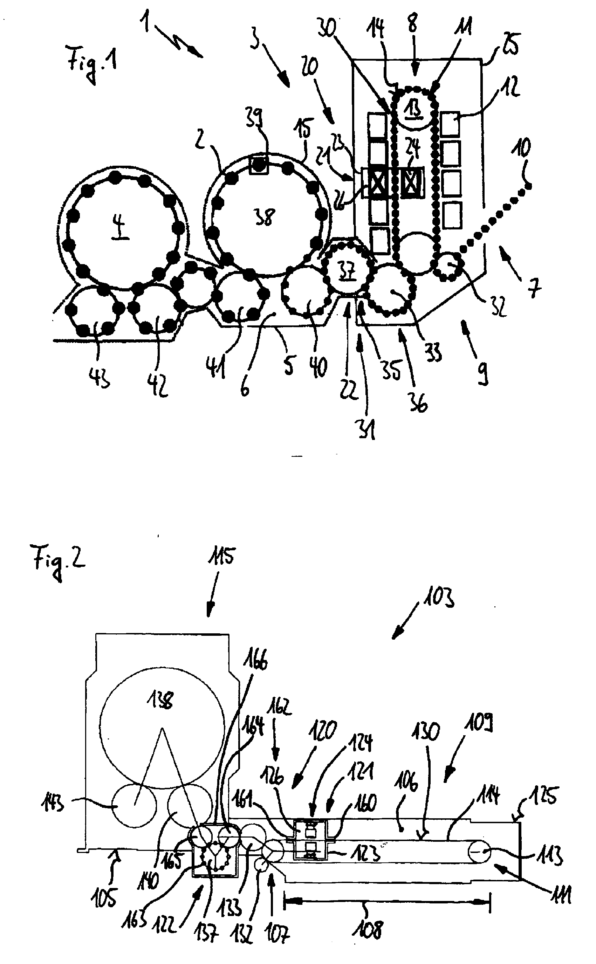

- Figur 1: schematisch eine Aufsicht einer ersten Getränkebehälterherstellanlage zum Herstellen von Kunststoffbehältnissen mit einer eine Zuführeinrichtung umfassenden Sterilisationseinrichtung mit Heizstreckentransportmitteln zum Transportieren der Kunststoffvorformlinge entlang einer Heizeinrichtungsheizstrecke; und
- Figur 2: schematisch eine Aufsicht einer weiteren Getränkebehälterherstellanlage zum Herstellen von Kunststoffbehältnissen mit einer eine Zuführeinrichtung umfassenden Sterilisationseinrichtung mit Heizstreckentransportmitteln zum Transportieren der Kunststoffvorformlinge entlang einer Heizeinrichtungsheizstrecke.

Die in der Figur 1 beispielhaft gezeigte Getränkebehälterherstellanlage 1 zum Herstellen von Kunststoffbehältnissen 2 weist im Wesentlichen eine Vorrichtung 3 zum Behandeln der Kunststoffbehältnisse 2 und eine daran anschließende Befüllungseinrichtung 4 auf, die in einem gemeinsamen Gehäuse 5 der Getränkebehälterherstellanlage 1 unter aseptischen Herstell- und Abfüllbedingungen in einem Reinraum 6 untergebracht sind.

Eingangsseitig 7 der Vorrichtung 3 ist eine eine Heizstrecke 8 umfassende Heizeinrichtung 9 vorgesehen, in der Kunststoffvorformlinge 10 erwärmt werden. Hierbei werden die Kunststoffvorformlinge 10 mittels einer Heizstreckentransporteinrichtung 11 durch die Heizeinrichtung 9 geführt und hierbei mit einer Vielzahl an Heizelementen 12 erwärmt. Die Heizstreckentransporteinrichtung 11 umfasst im Wesentlichen zwei Umlenkeinheiten 13 (nur exemplarisch beziffert) und Heizstreckentransportmittel 14, wie etwa eine umlaufende Kette. Zum Halten der einzelnen Kunststoffvorformlinge 10 weisen die Heizstreckentransportmittel 14 eine Vielzahl an an sich bekannten Dornhalteelementen (nicht gezeigt) auf, mittels welchen die jeweiligen Kunststoffvorformlinge 10 innenseitig am späteren Zugang bzw. Flaschenhalsbereich des Kunststoffbehältnisses 2 gehalten werden können. Dieses innenseitige Halten der Kunststoffvorformlinge 10 ermöglicht es, dass die Außenwandungsflächen der Kunststoffvorformlinge 10 vorteilhafter Weise nicht von irgendwelchen Halteelementen bedeckt und somit von außen vollständig frei zugänglich sind. Vorteilhafter Weise können die Kunststoffvorformlinge 10 hierdurch durch die Heizelemente 12 ohne Schattenbildung erwärmt werden.

An der Heizeinrichtung 9 und vor einer Umformeinrichtung 15, an welcher die Kunststoffvorformlinge 10 zu den Kunststoffbehältnissen 2 umgeformt werden, ist eine Sterilisationseinrichtung 20 vorgesehen. Die Sterilisationseinrichtung 20 umfasst eine Außenentkeimungseinheit 21 und eine Innenentkeimungseinheit 22, die voneinander getrennt an der Heizeinrichtung 9 angeordnet sind. Damit wird der eigentliche Sterilisationsbereich, der zum Abschirmen der schädlichen (Röntgen)strahlung mit einem eigenen Schutz versehen sein sollte, in zwei getrennte Teilbereiche, insbesondere einen Außensterilisationsbereich und einen Innensterilisationsbereich aufgetrennt, wobei diese beiden Teilbereiche vorteilhaft in der Transportrichtung der Behältnisse noch vor der Umformungseinrichtung (bei der es sich beispielsweise um eine Streckblasmaschine handeln kann) angeordnet sind.

Die Außenentkeimungseinheit 21 der Sterilisationseinrichtung 20 ist direkt in der Heizstrecke 8 zwischen zwei Heizelementen 12 integriert angeordnet, also somit auch innerhalb der Heizeinrichtung 9. Sie umfasst ein Außenentkeimungseinheitsgehäuse 23, in welchem eine Erzeugungseinrichtung 24 zum Erzeugen von elektrischen Ladungsteilchen platziert ist, wobei mittels der elektrischen Ladungsteilchen die Außenwandungsflächen der Kunststoffvorformlinge 10 unter aseptischen Bedingungen entkeimt werden können.

Insofern ist die Heizeinrichtung 9 mit der Erzeugungseinrichtung 24 zum Erzeugen der elektrischen Ladungsteilchen ausgerüstet, wodurch die Außenentkeimungseinheit 21 der Sterilisationseinrichtung 20 besonders gut in die Heizeinrichtung 9 integriert werden kann.

Um die Umwelt gegenüber den erzeugten elektrischen Ladungsteilchen in einem erforderlichen Maße gut zu schützen und insbesondere auch entstehende Röntgenstrahlung abzuschrimen, weist die Heizeinrichtung 9 zumindest teilweise ein ladungsteilchenabschirmendes Heizeinrichtungsgehäuse 25 auf.

Die aseptischen Bedingungen können speziell an der Außenentkeimungseinheit 21 besonders betriebssicher gewährleistet werden, wenn die Heizeinrichtung 9 noch einen zusätzlichen Sterilisationseinrichtungsreinraum 26 umfasst.

Die Sterilisationseinrichtung 20 umfasst eine Zuführeinrichtung 30, um der Außenentkeimungseinheit 21 die Kunststoffvorformlinge 10 zuzuführen, wobei die Zuführeinrichtung 30 der Sterilisationseinrichtung 20 erfindungsgemäß die mit den Dornhalteelementen ausgerüstete Heizstreckentransportmittel 14 umfasst, um die Kunststoffvorformlinge 10 konstruktiv besonders einfach zumindest der Außenentkeimungseinheit 21 der Sterilisationseinrichtung 20 zuzuführen. Insofern sind die Dornhalteelemente der Heizstreckentransportmittel 14 an der Außenentkeimungseinrichtung 21 temporär anordenbar (insbesondere, indem sie an diesen vorbeibewegt werden. Die Außenentkeimungseinheit 21 ist vorteilhaft stationär angeordnet.

Hieraus folgt auch, dass die Heizeinrichtung 9 vorteilhaft Mittel zum Drehen der Dornhalteelemente aufweist, mittels welchen die Kunststoffvorformlinge 10 zumindest an der Au-βenentkeimungseinrichtung 21 während einer Außensterilisation um eine Vertikalachse herum drehbar anordenbar sind.

Die Innenentkeimungseinheit 22 der Sterilisationseinrichtung 20 ist in diesem Ausführungsbeispiel am Ausgang 31 der Heizeinrichtung 9 angeordnet.

Insofern ist die Sterilisationseinrichtung 20 lediglich teilweise innerhalb der Heizeinrichtung 9 integriert, wobei die Außenentkeimungseinheit 21 innerhalb der Heizstrecke 8 und die Innenentkeimungseinheit 22 außerhalb der Heizstrecke 8 angeordnet sind.

Insofern kann insbesondere mit der vorliegenden Vorrichtung 3 ein Verfahren zum Umformen der Kunststoffvorformlingen 10 zu den Kunststoffbehältnissen 2 vorteilhaft realisiert werden, bei welchem die Kunststoffvorformlinge 10 mittels Druckluft zu den Kunststoffbehältnissen 2 expandiert werden, bei welchem die Kunststoffvorformlinge 10 vor dem Expandieren an der Heizstrecke 8 der Heizeinrichtung 9 vorgewärmt werden, und bei welchem die Kunststoffvorformlinge 10 mittels der Heizstreckentransportmittel 14 entlang der Heizstrecke 8 von einer Heizstreckenzufuhreinrichtung 32 zu einer Heizstreckenabfuhreinrichtung 33 transportiert werden, wobei die Kunststoffvorformlinge 10 an der Heizstrecke 8 zumindest teilweise sterilisiert werden, und wobei die an Halteelementen 12 der Heizstreckentransportmittel 14 gehaltenen Kunststoffvorformlinge 10 direkt an der Sterilisationseinrichtung 20 sterilisiert werden.

Die Heizstreckenabfuhreinrichtung 33 stellt eine Übergabeeinheit 36 dar, welche die Kunststoffvorformlinge 10 an die Innenentkeimungseinheit 22 der Sterilisationseinrichtung 20 übergibt. Diese Sterilisationseinrichtung 20 weist ein Transportrad 37 auf und an diesem Transportrad 37 oder auch stationär können Sterilisationselemente (hier nicht gezeigt) angeordnet sein. In diesem Bereich ist beispielsweise eine Sterilisation durch Wasserstoffperoxidgas oder auch, wie oben hinsichtlich der Außenentkeimungseinheit 21 erwähnt, durch Ladungsträger, insbesondere Elektronen, elektromagnetische Strahlung oder UV-Strahlung oder dergleichen und/oder Kombinationen hieraus möglich. Insbesondere wird in diesem Bereich aber die Innensterilisation der Kunststoffvorformlinge 10 durchgeführt.

Das Bezugszeichen 6 bezeichnet in seiner Gesamtheit den Reinraum, dessen Außenbegrenzungen durch das gemeinsame Gehäuse 5 angedeutet sind. In einer weiteren bevorzugten Ausführungsform ist der Reinraum 6 nicht nur im Bereich eines Transportrades 38 der Umformeinrichtung 15 und der Befüllungseinrichtung 4 angeordnet, sondern beginnt möglicherweise schon im Bereich der Heizeinrichtung 9, der Sterilisationseinrichtung 20, der Kunststoffvorformlingzuführung und/oder der Kunststoffvorformlingherstellung. Der Reinraum 6 ist an die Außengestalt der einzelnen Anlagenkomponenten angepasst. Auf diese Weise kann das Volumen des Reinraums 6 reduziert werden.

Das Bezugszeichen 15 bezeichnet in ihrer Gesamtheit eine Umformeinrichtung, bei der an dem Transportrad 38 eine Vielzahl von Blasstationen 39 angeordnet sind, wobei hier lediglich eine dieser Blasstationen 39 dargestellt ist. Mit diesen Blasstationen 39 werden die Kunststoffvorformlinge 10 zu den Kunststoffbehältnissen 2 expandiert.

Das Bezugszeichen 40 bezieht sich auf eine Zuführradeinrichtung, welche die Kunststoffvorformlinge 10 an die Umformeinrichtung 15 übergibt und das Bezugszeichen 41 bezieht sich auf eine Abführradeinrichtung, welche die hergestellten Kunststoffbehältnisse 2 von der Umformeinrichtung 15 abführt. Man erkennt, dass der Reinraum 6 in dem Bereich der Zuführeinrichtung 40 und der Abführeinrichtung 41 jeweils Ausnehmungen aufweist, welche diese Einrichtungen 40, 41 aufnehmen. Auf diese Weise kann in besonders vorteilhafter Weise eine Übergabe der Kunststoffvorformlinge 10 an die Umformeinrichtung 15 bzw. eine Übernahme der Kunststoffbehältnisse 2 von der Umformeinrichtung 15 erreicht werden.

Mit einer Übergabeeinheit 42 werden die expandierten Kunststoffbehältnisse 2 an die Befüllungseinrichtung 4 übergeben und von dieser Befüllungseinrichtung 4 anschließend über eine weitere Transporteinheit 43 abgeführt. Dabei befindet sich auch die Befüllungseinrichtung 4 innerhalb des besagten Reinraums 6, wie bereits beschrieben. Auch im Falle der Befüllungseinrichtung 4 wäre es möglich, dass nicht die gesamte Befüllungseinrichtung 4 mit beispielsweise einem Reservoir für ein Getränk vollständig innerhalb des Reinraums 6 angeordnet ist, sondern auch hier lediglich diejenigen Bereiche, in denen tatsächlich die Kunststoffbehältnisse 2 geführt werden. Insoweit könnte auch die Befüllungseinrichtung 4 in ähnlicher Weise aufgebaut sein wie die Umformeinrichtung 15 zum Umformen der Kunststoffvorformlinge 10. Bei den einzelnen Transport- bzw. Übergabeeinrichtungen handelt es sich bevorzugt um Transportsterne.

Bei dem in der Figur 2 gezeigten weiteren Ausführungsbeispiel ist eine andere Vorrichtung 103 zum Behandeln von Kunststoffbehältnissen (hier nicht gezeigt) im Wesentlichen mit einer Umformeinrichtung 115 zum Umformen von Kunststoffvorformlingen (hier nicht gezeigt) zu den nicht gezeigten Kunststoffbehältnissen, mit einer eine Heizstrecke 108 umfassenden Heizeinrichtung 109 zum Erwärmen der Kunststoffvorformlinge und mit einer Sterilisationseinrichtung 120 zum Sterilisieren der Kunststoffvorformlinge ausgestaltet.

Die Sterilisationseinrichtung 120 setzt sich aus einer Außenentkeimungseinheit 121 und einer Innenentkeimungseinheit 122 zusammen, wobei die Außenentkeimungseinrichtung 121 über eine Zuführeinrichtung 130 verfügt, um die Kunststoffvorformlinge der Sterilisationseinrichtung 120 zuzuführen, die sich erfindungsgemäß durch Heizstreckentransportmittel 114 einer Heizstreckentransporteinrichtung 111 der Heizeinrichtung 109 zum Transportieren der Kunststoffvorformlinge entlang der Heizstrecke 108 auszeichnet, um die Kunststoffvorformlinge somit baulich enorm einfach zumindest der Außenentkeimungseinheit 121 zuzuführen. Die Außenentkeimungseinheit 121 befindet sich in einem Reinraum 106 der insgesamt von einem gemeinsamen Gehäuse 105 der Vorrichtung 103 umhüllt ist. Die Außenentkeimungseinheit 121 weist darüber hinaus einen Sterilisationseinrichtungsreinraum 126 auf, der von einem Außenentkeimungseinheitsgehäuse 123 zusätzlich umgeben ist, und in welchem eine Erzeugungseinrichtung 124 zum Erzeugen von elektrischen Ladungsteilchen eingehaust ist. Das Außenentkeimungseinheitsgehäuse 123 gestaltet eine Abschirmeinrichtung gegenüber den Ladungsteilchen und/oder gegenüber entstehender Röntgenstrahlung. aus.

Durch diesen Sterilisationseinrichtungsreinraum 126 hindurch sind die Heizstreckentransportmittel 114 geführt, sodass daran gehalterte Kunststoffvorformlinge vorteilhafter Weise direkt an der Au βenentkeimungseinheit 120 sterilisiert werden können, wie dies bereits hinsichtlich des ersten Ausführungsbeispiels erläutert wurde.

Insofern gestalten die Heizstreckentransportmittel 114 auch in diesem zweiten Ausführungsbeispiel eine Zuführeinrichtung 130 der Sterilisationseinrichtung 120 zumindest hinsichtlich ihrer Außenentkeimungseinheit 121 direkt aus.

Zum Führen der Heizstreckentransportmittel 114 durch die Außenentkeimungseinheit 120 hindurch, weist die Außenentkeimungseinheit 120 eine Eingangsöffnung 160 und eine Ausgangsöffnung 161 auf, die sich beide innerhalb eines Heizungsgehäuses 125 der Heizeinrichtung 109 befinden.

Vorteilhafter Weise sind die Kunststoffvorformlinge mit Dornhalteelementen innen gehalten, sodass sie an der Außenentkeimungseinheit 120 außen auch gut entkeimt werden können.

Die Außenentkeimungseinheit 120 ist hierbei am Ende 162 der Heizstrecke 108 und vor einer Heizstreckenabfuhreinrichtung 133 platziert. Auch ist die Außenentkeimungseinrichtung 120 zwischen zwei Umlenkeinheiten 113 zum Umlenken der Heizstreckentransportmittel 114 gelagert, wobei eine der Umlenkeinheiten 113 eingangsseitig 107 der Heizeinrichtung 109 sowohl mit einer Heizstreckenzufuhreinrichtung 132 als auch mit einer Heizstreckenabfuhreinrichtung 133 direkt wechselwirkt.

Die Innenentkeimungseinrichtung 122 der Sterilisationseinrichtung 120 ist funktional zwischen der Heizstreckenabfuhreinrichtung 133 und einer Zuführradeinrichtung 140 platziert und verfügt über ein Transportrad 137 und über an sich bekannte Innensterilisationselemente mit Strahlfingerelementen 163, mittels welchen die Innenwandungsflächen der Kunststoffvorformlinge entkeimt werden können. Das Transportrad 137 kommuniziert mit zwei Transfersterneinrichtungen 164 und 165, mittels welchen die Kunststoffvorformlinge in die Innenentkeimungseinrichtung 122 hinein bzw. aus der Innenentkeimungseinrichtung 122 heraus geschleust werden. Das Transportrad 137, die Strahlfingerelemente 163 und die Transfersterneinrichtungen 164, 165 sind hierbei in einem Innenentkeimungseinheitsgehäuse 166 zusätzlich geschützt angeordnet. Das Innenentkeimungseinheitsgehäuse 166 gestaltet sogleich eine Abschirmeinrichtung gegenüber den Ladungsteilchen und/oder gegenüber Röntgenstrahlung aus.

Vorzugsweise werden die Behältnisse gegenüber den Strahlfingerelementen 163 in einer Längsrichtung der Behältnisse bewegt, um die Strahlfingerelemente, an deren unteren Enden sich bevorzugt Austrittsfenster für die Elektronen befinden, in die Behältnisse einzuführen. Bevorzugt werden hierbei die Behältnisse in ihrer Längsrichtung bewegt, es wäre jedoch auch möglich, die Strahlfingerelemente 163 zu bewegen oder auch, sowohl die Strahlfingerelemente als auch die Behältnisse zu bewegen.

Die beiden hier beschriebenen Teilbereiche zur Außen- und Innenentkeimung der Vorformlinge sind bevorzugt Teil der aseptischen Einhausung der Gesamtanlage und sollten bevorzugt nach hygienischen Gesichtspunkten gestaltet sein. Bevorzugt ist daher ebenfalls zumindest ein Teilbereich der Erwärmungsvorrichtung nach hygienischen Gesichtspunkten aufgebaut. Zwischen den beiden Entkeimungsbereichen können herkömmliche Einhausungen verwendet werden, da die Strahlung nur jeweils unmittelbar an den Emittern abgeschirmt werden muss.

An der Umformungseinrichtung 115 werden die so sterilisierten und mittels der Zuführradeinrichtung 140 einem Blasrad 138 zugeführten Kunststoffvorformlinge zu den Kunststoffbehältnissen expandiert, bevor sie dann zur weiteren Verwendung an einer Transporteinheit 143 bereitgestellt werden.

Es versteht sich, dass es sich bei den vorstehend erläuterten Ausführungsbeispielen lediglich um erste Ausgestaltungen der erfindungsgemäßen Vorrichtung zum Behandeln von Kunststoffbehältnissen handelt. Insofern beschränken sich die Ausgestaltungen der Erfindung nicht auf diese Ausführungsbeispiele.

### Bezugszeichenliste

- 1: Getränkebehälterherstellanlage
- 2: Kunststoffbehältnisse
- 3: Vorrichtung zum Behandeln
- 4: Befüllungseinrichtung
- 5: gemeinsames Gehäuse
- 6: Reinraum
- 7: eingangsseitig
- 8: Heizstrecke
- 9: Heizeinrichtung
- 10: Kunststoffvorformlinge
- 11: Heizstreckentransporteinrichtung
- 12: Heizelemente
- 13: Umlenkeinheiten
- 14: Heizstreckentransportmittel
- 15: Umformeinrichtung
- 20: Sterilisationseinrichtung
- 21: Außenentkeimungseinheit
- 22: Innenentkeimungseinheit
- 23: Außenentkeimungseinheitsgehäuse
- 24: Erzeugungseinrichtung
- 25: Heizeinrichtungsgehäuse
- 26: Heizeinrichtungsreinraum
- 30: Zuführeinrichtung
- 31: Ausgang
- 32: Heizstreckenzufuhreinrichtung
- 33: Heizstreckenabfuhreinrichtung
- 36: Übergabeeinheit
- 37: Transportrad
- 38: Blasrad
- 39: Blasstationen
- 40: Zuführradeinrichtung
- 41: Abführradeinrichtung
- 42: Übergabeeinheit
- 43: Transporteinheit
- 103: Vorrichtung zum Behandeln
- 105: gemeinsames Gehäuse
- 106: Reinraum
- 107: eingangsseitig
- 108: Heizstrecke
- 109: Heizeinrichtung
- 111: Heizstreckentransporteinrichtung
- 113: Umlenkeinheiten
- 114: Heizstreckentransportmittel
- 115: Umformeinrichtung
- 120: Sterilisationseinrichtung
- 121: Außenentkeimungseinheit
- 122: Innenentkeimungseinheit
- 123: Außenentkeimungseinheitsgehäuse
- 124: Erzeugungseinrichtung
- 125: Heizeinrichtungsgehäuse
- 126: Sterilisationseinrichtungsreinraum
- 130: Zuführeinrichtung
- 132: Heizstreckenzufuhreinrichtung
- 133: Heizstreckenabfuhreinrichtung
- 137: Transportrad
- 138: Blasrad
- 140: Zuführradeinrichtung
- 143: Transporteinheit
- 160: Eingangsöffnung
- 161: Ausgangsöffnung
- 162: Ende
- 163: Strahlfingerelemente
- 164: erste Transfersterneinrichtung
- 165: zweite Transfersterneinrichtung
- 166: Innenentkeimungseinheitsgehäuse

## Patentansprüche

1. Vorrichtung (3; 103) zum Behandeln von Kunststoffbehältnissen (2) mit einer Umformeinrichtung (15; 115) zum Umformen von Kunststoffvorformlingen (10) zu den Kunststoffbehältnissen (2), mit einer eine Heizstrecke (8; 108) umfassenden Heizeinrichtung (9; 109) zum Erwärmen der Kunststoffvorformlinge (10) und mit einer Sterilisationseinrichtung (20; 120) zum Sterilisieren der Kunststoffvorformlinge (10), bei welcher die Sterilisationseinrichtung (20; 120) eine Zuführeinrichtung (30, 130) umfasst, um die Kunststoffvorformlinge (10) der Sterilisationseinrichtung (20; 120) zuzuführen, und bei welcher die Sterilisationseinrichtung (20; 120) sowohl eine Außenentkeimungseinheit (21; 121), um die Kunststoffvorformlinge (10) zumindest an ihren Außenwandungsflächen zu sterilisieren, als auch eine Innenentkeimungseinheit (22; 122), um die Kunststoffvorformlinge (10) an ihren Innenwandungsflächen zu sterilisieren, umfasst,
wobei
die Zuführeinrichtung (30; 130) Heizstreckentransportmittel (14; 114) zum Transportieren der Kunststoffvorformlinge (10) entlang der Heizstrecke (8; 108) umfasst, um die Kunststoffvorformlinge (10) zumindest der Außenentkeimungseinheit (21; 121) zuzuführen und die Sterilisationseinrichtung (20; 120) lediglich teilweise innerhalb der Heizeinrichtung (9; 109) integriert ist, wobei die Außenentkeimungseinheit (21; 121) innerhalb der Heizstrecke (8; 108) und die Innenentkeimungseinheit (22; 122) außerhalb der Heizstrecke (8; 108) und außerhalb der Heizeinrichtung (9; 109) angeordnet sind, **dadurch gekennzeichnet, dass** die Innenentkeimungseinheit (22; 122) am Ausgang (31) der Heizeinrichtung (9; 109) angeordnet ist.

2. Vorrichtung (3; 103) nach Anspruch 1,
**dadurch gekennzeichnet, dass**
die Heizstreckentransportmittel (14; 114) eine Vielzahl an Halteelementen zum Halten der Kunststoffvorformlinge (10), insbesondere Dornhalteelemente zum innenseitigen Halten der Kunststoffvorformlinge (10), umfassen, wobei die Halteelemente bzw. die Dornhalteelemente der Heizstreckentransportmittel (14; 114) an der Außenentkeimungseinrichtung (21; 121) temporär anordenbar sind.

3. Vorrichtung (3; 103) nach einem der Ansprüche 1 bis 2,
**dadurch gekennzeichnet, dass**
die Außenentkeimungseinheit (21; 121) am Anfang der Heizstrecke (8; 108) vor einem Heizelement (12) und nach einem Eingang (7; 107) der Heizeinrichtung (9; 109), zwischen zwei Heizelementen (12) innerhalb der Heizstrecke (8; 108) oder am Ende der Heizstrecke (8; 108) nach einem Heizelement (12) und vor einer Übergabeeinrichtung (33; 133) am Ausgang (31) und innerhalb der Heizeinrichtung (9; 109) angeordnet ist.

4. Vorrichtung (3; 103) nach einem der Ansprüche 1 bis 3,
**dadurch gekennzeichnet, dass**
die Heizeinrichtung (9; 109) Mittel zum Drehen der Halte- oder Dornhalteelemente aufweist, mittels welchen die Kunststoffvorformlinge (10) zumindest an der Außenentkeimungseinrichtung (21; 121) während einer Außensterilisation um eine Vertikalachse herum drehbar anordenbar sind.

5. Vorrichtung (3; 103) nach einem der Ansprüche 1 bis 4,
**dadurch gekennzeichnet, dass**
die Heizeinrichtung (9; 109) eine Erzeugungseinrichtung (24;124) zum Erzeugen von elektrischen Ladungsteilchen umfasst, mittels welchen die Kunststoffvorformlinge (10) sterilisierbar sind.

6. Vorrichtung (3; 103) nach einem der Ansprüche 1 bis 5,
**dadurch gekennzeichnet, dass**
die Heizeinrichtung (9; 109) zumindest teilweise ein ladungsteilchenabschirmendes und/oder röntgenstrahlungabschirmendes Gehäuse (25;125) umfasst.

7. Vorrichtung (3; 103) nach einem der Ansprüche 1 bis 6,
**dadurch gekennzeichnet, dass**
die Heizeinrichtung (9; 109) einen zusätzlichen Reinraum (26; 126) umfasst, in welchem die Sterilisationseinrichtung (20; 120) zumindest teilweise angeordnet ist.

8. Vorrichtung (3; 103) nach einem der Ansprüche 1 bis 7,
**dadurch gekennzeichnet, dass**
eine Innenentkeimungseinheit (22; 122) der Sterilisationseinrichtung (20; 120) außerhalb der Heizeinrichtung (9; 109) und damit beabstandet von einer innerhalb der Heizeinrichtung (9; 109) angeordneten Außenentkeimungseinheit (21; 121) der Sterilisationseinrichtung (20; 120) angeordnet ist, wobei insbesondere sowohl die Innenentkeimungseinheit (22; 122) als auch die Außenentkeimungseinheit (21; 121) innerhalb eines gemeinsamen Reinraums (6; 106) unter aseptischen Bedingungen angeordnet sind.

9. Getränkeabfüllanlage und/oder Getränkebehälterherstellanlage (1) mit einer Vorrichtung (3; 103) zum Umformen von Kunststoffvorformlingen (10) zu Kunststoffbehältnissen (2),
**gekennzeichnet**
**durch** eine Vorrichtung (3; 103) nach einem der vorhergehenden Ansprüchen.

10. Verfahren zum Umformen von Kunststoffvorformlingen (10) zu Kunststoffbehältnissen (2), bei welchem die Kunststoffvorformlinge (10) mittels Druckluft zu den Kunststoffbehältnissen (2) expandiert werden, bei welchem die Kunststoffvorformlinge (10) vor dem Expandieren an einer Heizstrecke (8; 108) einer Heizeinrichtung (9; 109) vorgewärmt werden, und bei welchem die Kunststoffvorformlinge (10) mittels Heizstreckentransportmittel (14; 114) entlang der Heizstrecke (8; 108) von einer Heizstreckenzufuhreinrichtung (32; 132) zu einer Heizstreckenabfuhreinrichtung (33; 133) transportiert werden, wobei
die Kunststoffvorformlinge (10) an der Heizstrecke (8; 108) zumindest teilweise sterilisiert werden, wobei die an Halteelementen der Heizstreckentransportmittel (14; 114) gehaltenen Kunststoffvorformlinge (10) direkt an einer Sterilisationseinrichtung (20; 120) sterilisiert werden und die Sterilisationseinrichtung (20; 120) eine Außenentkeimungseinheit (21; 121) und eine Innenentkeimungseinheit (22; 122) umfasst und lediglich teilweise innerhalb der Heizeinrichtung (9; 109) integriert ist, wobei die Kunststoffvorformlinge (10) innerhalb der Heizstrecke (8; 108) durch die Außenentkeimungseinheit (21; 121) außensterilisiert werden, **dadurch gekennzeichnet, dass** die Kunststoffvorformlinge (10) außerhalb der Heizstrecke (8; 108) und Heizeinrichtung (9; 109) durch die am Ausgang (31) der Heizeinrichtung (9; 109) angeordneten Innenentkeimungseinheit (22; 122) innensterilisiert werden.

11. Verfahren nach Anspruch 10,
**dadurch gekennzeichnet, dass**
die Kunststoffvorformlinge (10) innerhalb der Heizstrecke (8; 108) zum Sterilisieren zumindest teilweise mit Ladungsträgern bestrahlt werden.

## Claims

1. An apparatus (3; 103) for treating plastics material containers (2) with a shaping device (15; 115) for shaping the plastics material pre-forms (10) into the plastics material containers (2), with a heating device (9; 109) comprising a heating path (8; 108) for heating the plastics material pre-forms (10) and with a sterilization device (20; 120) for the sterilization of the plastics material pre-forms (10), in which the sterilization device (20; 120) comprises a supply device (30, 130) in order to supply the plastics material preforms (10) to the sterilization device (20; 120), and in which the sterilization device (20; 120) comprises both an external disinfecting unit (21; 121) in order to sterilize the plastics material pre-forms (10) at least on their external wall surfaces and an internal disinfecting unit (22; 122) in order to sterilize the plastics material pre-forms (10) on their internal wall surfaces, wherein the supply device (30, 130) comprises heating path conveying means (14; 114) for conveying the plastics material pre-forms (10) along the heating path (8; 108), in order to supply the plastics material pre-forms (10) at least to the external disinfecting unit (21; 121) and the sterilization device (20; 120) is integrated solely partially in the interior of the heating device (9; 109), the external disinfecting unit (21; 121) being arranged in the interior of the heating path (8; 108) and the internal disinfecting unit (22; 122) being arranged outside of the heating path (8; 108) and outside of the heating device (9; 109), **characterized in that** the internal disinfecting unit (22; 122) is arranged at the outlet (31) of the heating device (9; 109).

2. An apparatus (3; 103) according to claim 1, **characterized in that** the heating path conveying means (14; 114) comprise a plurality of holding elements for holding the plastics material pre-forms (10), in particular mandrel-type holding elements for holding the plastics material pre-forms (10) on the inside, wherein the holding elements or the mandrel-type holding elements respectively of the heating path conveying means (14; 114) are capable of being arranged temporarily on the external disinfection device (21; 121).

3. An apparatus (3; 103) according to any one of claims 1 to 2, **characterized in that** the external disinfection unit (21; 121) is arranged at the start of the heating path (8; 108) upstream of a heating element (12) and downstream of an inlet (7; 107) of the heating device (9; 109), between two heating elements (12) inside the heating path (8; 108) or at the end of the heating path (8; 108) downstream of a heating element (12) and upstream of a transfer device (33; 133) at the outlet (31) and inside the heating device (9; 109).

4. An apparatus (3; 103) according to any one of claims 1 to 3, **characterized in that** the heating device (9; 109) has means for rotating the holding elements or mandrel-type holding elements, by means of which the plastics material pre-forms (10) are capable of being arranged at least on the external disinfection device (21; 121) so as to be rotatable about a vertical axis during an external sterilization.

5. An apparatus (3; 103) according to any one of claims 1 to 4, **characterized in that** the heating device (9; 109) comprises a generation device (24; 124) for the generation of electrical charge particles, by means of which the plastics material pre-forms (10) are capable of being sterilized.

6. An apparatus (3; 103) according to any one of claims 1 to 5, **characterized in that** the heating device (9; 109) comprises at least in part a casing (25; 125) which screens out the charge particles and/or screens out X-ray radiation.

7. An apparatus (3; 103) according to any one of claims 1 to 6, **characterized in that** the heating device (9; 109) comprises an additional clean room (26; 126) in which the sterilization device (20; 120) is arranged at least in part.

8. An apparatus (3; 103) according to any one of claims 1 to 7, **characterized in that** an internal disinfection unit (22; 122) of the sterilization device (20; 120) is arranged outside the heating device (9; 109) and thus at a distance from an external disinfection unit (21; 121) of the sterilization device (20; 120) arranged inside the heating device (9; 109), wherein in particular both the internal disinfection unit (22; 122) and the external disinfection unit (21; 121) are arranged inside a common clean room (6; 106) under aseptic conditions.

9. A beverage filling plant and/or a beverage container production plant (1) with an apparatus (3; 103) for shaping plastics material pre-forms (10) into plastics material containers (2), **characterized by** an apparatus (3; 103) according to any one of the preceding claims.

10. A method of shaping plastics material pre-forms (10) into plastics material containers (2), in which the plastics material pre-forms (10) are expanded by means of compressed air to form the plastics material containers (2), in which before the expansion the plastics material pre-forms (10) are pre-heated on a heating path (8; 108) of a heating device (9; 109), and in which the plastics material pre-forms (10) are conveyed by means of heating path conveying means (14; 114) along the heating path (8; 108) from a heating path supply device (32; 132) to a heating path removal device (33; 133), wherein the plastics material pre-forms (10) are sterilized at least in part on the heating path (8; 108), wherein the plastics material pre-forms (10) held on holding elements of the heating path conveying means (14; 114) are sterilized directly on a sterilization device (20; 120) and the sterilization device (20; 120) comprises an external disinfecting unit (21; 121) and an internal disinfecting unit (22; 122) and is integrated solely partially in the interior of the heating device (9; 109), the plastics material preforms (10) being sterilized at the outside by the external disinfecting unit (21; 121) in the interior of the heating path (8; 108), **characterized in that** the plastics material preforms (10) are sterilized at the inside by the internal disinfecting unit (22; 122) being arranged at the outlet (31) of the heating device (9; 109) outside of the heating path (8; 108) and the heating device (9; 109).

11. A method according to claim 10, **characterized in that** the plastics material pre-forms (10) are irradiated at least in part with charge carriers inside the heating path (8; 108) for sterilization purposes.

## Revendications

1. Dispositif (3 ; 103) pour le traitement de récipients en matière plastique (2) par un dispositif de transformation (15 ; 115) pour transformer des préformes en matière plastique (10) en lesdits récipients en matière plastique (2), avec un dispositif de chauffage (9 ; 109) comportant un trajet de chauffe (8 ; 108) pour le chauffage des préformes en matière plastique (10), et avec un dispositif de stérilisation (20 ; 120) pour la stérilisation des préformes en matière plastique (10), où le dispositif de stérilisation (20 ; 120) comprend un dispositif d'amenée (30, 130) pour conduire les préformes en matière plastique (10) vers le dispositif de stérilisation (20 ; 120), et où le dispositif de stérilisation (20 ; 120) comprend une unité de désinfection extérieure (21 ; 121) pour stériliser les préformes en matière plastique (10) au moins sur leurs surfaces de paroi extérieure ainsi qu'une unité de désinfection intérieure (22 ; 122) pour stériliser les préformes en matière plastique (10) sur leurs surfaces de paroi intérieure,
le dispositif d'amenée (30 ; 130) comprenant des moyens de transport (14 ; 114) sur le trajet de chauffe, pour le transport des préformes en matière plastique (10) le long du trajet de chauffe (8 ; 108), pour conduire les préformes en matière plastique (10) au moins vers l'unité de désinfection extérieure (21 ; 121) et le dispositif de stérilisation (20 ; 120) n'étant intégré que partiellement à l'intérieur du dispositif de chauffage (9 ; 109), l'unité de désinfection extérieure (21 ; 121) étant située à l'intérieur du trajet de chauffe (8 ; 108), et l'unité de désinfection intérieure (22 ; 122) à l'extérieur du trajet de chauffe (8 ; 108) et à l'extérieur du dispositif de chauffage (9 ; 109), **caractérisé en ce que** l'unité de désinfection intérieure (22 ; 122) est située à la sortie (31) du dispositif de chauffage (9 ; 109).

2. Dispositif (3 ; 103) selon la revendication 1, **caractérisé en ce que** les moyens de transport sur le trajet de chauffe (14 ; 114) comprennent une pluralité d'éléments de retenue pour le maintien des préformes en matière plastique (10), en particulier des éléments de retenue à mandrin pour le maintien par l'intérieur des préformes en matière plastique (10), les éléments de retenue et respectivement les éléments de retenue à mandrin des moyens de transport (14 ; 114) sur le trajet de chauffe pouvant être temporairement disposés sur l'unité de désinfection extérieure (21 ; 121).

3. Dispositif (3 ; 103) selon l'une des revendications 1 ou 2, **caractérisé en ce que**
l'unité de désinfection extérieure (21 ; 121) est située au début du trajet de chauffe (8 ; 108) en amont d'un élément de chauffe (12) et en aval d'une entrée (7 ; 107) du dispositif de chauffage (9 ; 109), entre deux éléments de chauffe (12) à l'intérieur du trajet de chauffe (8 ; 108), ou à l'extrémité du trajet de chauffe (8 ; 108) en aval d'un élément de chauffe (12) et en amont d'un dispositif de transfert (33 ; 133) à la sortie (31) et à l'intérieur du dispositif de chauffage (9 ; 109).

4. Dispositif (3 ; 103) selon l'une des revendications 1 à 3, **caractérisé en ce que**
le dispositif de chauffage (9 ; 109) comporte des moyens de rotation des éléments de retenue ou des éléments de retenue à mandrin, au moyen desquels les préformes en matière plastique (10) peuvent être disposés de manière rotative tout autour d'un axe vertical pendant une stérilisation extérieure au moins sur l'unité de désinfection extérieure (21 ; 121).

5. Dispositif (3 ; 103) selon l'une des revendications 1 à 4, **caractérisé en ce que**
le dispositif de chauffage (9 ; 109) comprend un dispositif de génération (24 ; 124) pour la génération de particules électriques de chargement au moyen desquelles les préformes en matière plastique (10) peuvent être stérilisées.

6. Dispositif (3 ; 103) selon l'une des revendications 1 à 5, **caractérisé en ce que**
le dispositif de chauffage (9 ; 109) comprend au moins en partie un carter (25 ; 125) protecteur contre les particules de chargement et/ou contre les rayons X.

7. Dispositif (3 ; 103) selon l'une des revendications 1 à 6, **caractérisé en ce que**
le dispositif de chauffage (9 ; 109) comprend un espace stérile (26 ; 126) complémentaire où le dispositif de stérilisation (20 ; 120) est situé au moins en partie.

8. Dispositif (3 ; 103) selon l'une des revendications 1 à 7, **caractérisé en ce qu'**
une unité de désinfection intérieure (22 ; 122) du dispositif de stérilisation (20 ; 120) est située à l'extérieur du dispositif de chauffage (9 ; 109) et est par conséquent espacée d'une unité de désinfection extérieure (21 ; 121) du dispositif de stérilisation (20 ; 120) située à l'intérieur du dispositif de chauffage (9 ; 109), l'unité de désinfection intérieure (22 ; 122) ainsi que l'unité de désinfection extérieure (21 ; 121) étant en particulier situées dans des conditions aseptiques à l'intérieur d'un espace stérile (6 ; 106) commun.

9. Installation de soutirage de boissons et/ou installation de fabrication de récipients pour des boissons (1) avec un dispositif (3 ; 103) de transformation de préformes en matière plastique (10) en récipients en matière plastique (2),
**caractérisée par** un dispositif (3 ; 103) selon l'une des revendications précédentes.

10. Procédé de transformation de préformes en matière plastique (10) en récipients en matière plastique (2), où les préformes en matière plastique (10) sont expansées au moyen d'air comprimé pour former lesdits récipients en matière plastique (2), où les préformes en matière plastique (10) sont pré-chauffées avant l'expansion sur un trajet de chauffe (8 ; 108) d'un dispositif de chauffage (9 ; 109), et où les préformes en matière plastique (10) sont transportées par des moyens de transport (14 ; 114) le long du trajet de chauffe (8 ; 108), d'un dispositif d'amenée (32 ; 132) au trajet de chauffe à un dispositif de sortie (33 ; 133) du trajet de chauffe,
les préformes en matière plastique (10) étant au moins en partie stérilisées sur le trajet de chauffe (8 ; 108), les préformes en matière plastique (10) maintenues sur des éléments de retenue des moyens de transport (14 ; 114) étant directement stérilisées sur un dispositif de stérilisation (20 ; 120), et le dispositif de stérilisation (20 ; 120) comprenant une unité de désinfection extérieure (21 ; 121) et une unité de désinfection intérieure (22 ; 122) et n'étant intégré que partiellement à l'intérieur du dispositif de chauffage (9 ; 109), les préformes en matière plastique (10) étant stérilisées extérieurement par l'unité de désinfection extérieure (21 ; 121) à l'intérieur du trajet de chauffe (8 ; 108), **caractérisé en ce que** les préformes en matière plastique (10) sont stérilisées intérieurement par l'unité de désinfection intérieure (22 ; 122) située à la sortie (31) du dispositif de chauffage (9 ; 109), à l'extérieur du trajet de chauffe (8 ; 108) et du dispositif de chauffage (9 ; 109).

11. Procédé selon la revendication 10, **caractérisé en ce que**
les préformes en matière plastique (10) sont irradiées par des porteurs de charge pour être stérilisées au moins en partie à l'intérieur du trajet de chauffe (8 ; 108).
